# EUROPÄISCHE PATENTSCHRIFT

(11) **EP 0 805 141 B1**
(45) Veröffentlichungstag und Bekanntmachung des Hinweises auf die Patenterteilung: **01.12.1999**
(21) Anmeldenummer: 97104264.3
(22) Anmeldetag: 13.03.1997
(51) Int. Cl.: C07C 67/08, C07C 69/63

(54) **Verfahren zur kontinuierlichen Herstellung von Estern thermisch labiler Säuren**
Process for the preparation of esters of thermically unstable acids
Procédé de préparation continue d'esters d'acides thermiquement instable

(30) Priorität: 04.05.1996 DE 19617991
(43) Veröffentlichungstag der Anmeldung: 05.11.1997
(73) Patentinhaber: Degussa-Hüls Aktiengesellschaft, 60287 Frankfurt am Main (DE)
(72) Erfinder: Kaufhold, Manfred, Dr., 45770 Marl (DE)
(74) Vertreter: Olbricht, Gerhard, Dr.

(56) Entgegenhaltungen:
- DE-A- 1 939 759

## Beschreibung

Verschiedene Ester der Cyanessigsäure, 4-Chlorbuttersäure, Thioglykolsäure und Malonsäure sind wichtige Zwischenprodukte für Herstellung anderer ' wertvoller Stoffe. So braucht man Cyanessigsäureester für Synthesen von Heterocyclen. 4-Chlorbuttersäureester dient als Ausgangsstoff für die Synthese von Cyclopropanderivaten. Für die Herstellung der benötigten technischen Mengen an diesen und anderen Estern der Säuren ist ein kontinuierliches Veresterungsverfahren erwünscht.

Dabei scheiden Veresterungsverfahren, die in der Gasphase arbeiten (siehe z.B. Ullmann, Enzyklopädie der technischen Chemie. 3. Auflage. 1967. Band 18, Seite 69), wegen der thermischen Empfindlichkeit der Sauren aus. In DD 127 l17 ist ein in flüssiger Phase arbeitendes kontinuierliches Verfahren beschrieben. Dabei wird die Säure in Gegenwart eines Katalysators in drei aufeinanderfolgenden Stufen verestert, und zwar in der ersten Stufe im Gleichstrom, in der zweiten Stufe im Gegenstrom und in der dritten Stufe wieder im Gleichstrom. Das Verfahren benötigt ein zusätzliches Schleppmittel für die Ausschleusung des Reaktionswassers, ist wegen seiner drei Veresterungsstufen apparativ aufwendig. erfordert wegen der unterschiedlichen Parameter in den drei Veresterungsstufen erheblichen meß- und regeltechnischen Aufwand und ist dementsprechend störanfällig. Bei der Herstellung von Cyanessigsäureestern betragen die Temperaturen in den drei Veresterungsstufen 75°C (Kolonne 4), 103°C (Kolonne 1) bzw. 110°C (Kolonne 2), und die entsprechenden Umsätze 10 bis 15%. 70 bis 80% bzw. wieder 10 bis 15%. Diese Verfahrensführung trägt der Tatsache Rechnung. daß Cyanessigsäure sich schon ab ca. 80°C zu zersetzen beginnt. Dementsprechend wird die Temperatur schrittweise gesteigert. und die Endtemperatur wird erst erreicht, wenn nur noch wenig unveresterte Cyanessigsäure im Reaktionsgemisch vorliegt.

Es wurde nun gefunden, daß sich Ester der Cyanessigsäure, 4-Chlorbuttersäure, Thioglykolsäure und Malonsäure durch Veresterung der Säuren mit Alkoholen vorteilhaft herstellen lassen, wenn man die Veresterung bei Temperaturen von 80 °C bis 130 °C in flüssiger Phase im wesentlichen in nur einer Reaktionszone durchführt, der man die Säure und einen Überschuß an Alkohol kontinuierlich zuführt und aus der man Alkohol zusammen mit Reaktionswasser in Dampfform sowie ein an Ester reiches Reaktionsgemisch in flüssiger Form kontinuierlich abzieht und aus diesem den Ester gewinnt.

Das Verfahren ergibt trotz der verhältnismäßig hohen Temperaturen und der erheblichen Verweilzeiten der Säuren in der Reaktionszone sehr gute Ausbeuten an den jeweiligen Estern. Das Verfahren ist apparativ vergleichsweise einfach, erfordert einen geringeren meß und regeltechnischen Aufwand als das bekannte kontinuierliche Verfahren und ist daher weniger störanfällig. Schleppmittel für das Reaktionswasser oder andere Hilfsstoffe werden nicht benötigt.

Besonders vorteilhaft läßt sich 4-Chlorbuttersäure nach dem Verfahren der Erfindung verestern.

Nach dem Verfahren der Erfindung werden vorteilhaft Ester mit Alkoholen hergestellt, die einen niedrigeren Siedepunkt als die betreffende Säure haben. Geeignete Alkohole sind insbesondere Alkanole mit 1 bis 3 Kohlenstoffatomen, also Methanol, Ethanol, n- und Isopropanol.

Es ist ein wichtiges Merkmal der Erfindung, daß die Veresterung in flüssiger Phase und, anders als bei dem Verfahren nach DD 127 117, im wesentlichen in nur einer Reaktionszone durchgeführt wird. "Im wesentlichen" bedeutet, daß die Veresterung in geringem Umfang auch an einem anderen Ort, z.B. im Rücklauf der Kolonne stattfinden kann, über die man zweckmäßig Alkohol und Wasser in Dampfform abzieht. Dabei wird die zu veresternde Säure, ihrem Dampfdruck entsprechend, mit ausgetragen und kann im Rücklauf, der überwiegend Alkohol enthält, in geringem Umfang verestert werden. Auf jeden Fall wird jedoch die weit über'wiegende Menge des Esters in der eigentlichen Reaktionszone gebildet.

Die Veresterung kann mit oder ohne einen der bekannten Veresterungskatalysatoren durchgeführt werden. Besonders geeignet, weil gut dosierbar, leicht löslich im Reaktionsgemisch und gut wirksam, sind C₁₀-C₁₃-Alkylbenzolsulfonsäuren. Andere geeignete Veresterungskatalysatoren sind z.B. Schwefelsäure, Methan- oder andere Alkansulfosäuren sowie p-Toluolsulfonsäure. Man wendet die Veresterungskatalysatoren zweckmäßig in Mengen von etwa 0,1 bis 1,0 Gewichtsprozent an, bezogen auf die eingesetzte thermisch labile Säure.

Die Temperatur in der Reaktionszone liegt im Bereich von 80 °C bis 130 °C. Die optimale Temperatur hängt in erster Linie von der jeweiligen Säure sowie auch davon ab, ob mit oder ohne Veresterungskatalysator gearbeitet wird. Sie läßt sich durch Routineversuche leicht ermitteln. Durch Mitverwendung eines Katalysators lassen sich schon bei niedrigen Temperaturen akzeptable Raum-Zeit-Ausbeuten erzielen. Für Cyanessigsäure mit Katalysator liegt die optimale Veresterungstemperatur bei 100 °C bis 120 °C, insbesondere bei 105°C bis 115°C. Bei 4-Chlorbuttersäure ohne Katalysator werden 80 °C bis 100 °C und insbesondere 85 °C bis 95 °C bevorzugt.

Der Reaktionszone werden die zu veresternde Säure, ein Überschuß an Alkohol und gegebenenfalls ein Katalysator kontinuierlich zugeführt. Das Molverhältnis von Alkohol zu Säure beträgt im allgemeinen 15:1 bis 3:1, vorteilhaft 10:1 bis 4:1 und insbesondere 8:1 bis 5:1. Man kann Säure, Alkohol und gegebenenfalls Katalysator jeweils einzeln, in Zweierkombinationen oder gemeinsam zuführen. Bei einer zweckmäßigen Ausführungsform des Verfahrens der Erfindung wird der Alkohol, der zusammen mit dem Reaktionswasser aus der Reaktionszone entfernt wird, nach Abtrennung vom Reaktionswasser in die Reaktionszone zurückgeführt. Zweckmäßig führt man die genannten Stoffe entfernt und, je nach der Geometrie der Reaktionszone, sogar möglichst weit entfernt von der Stelle zu, an der an Ester reiches flüssiges Produkt aus der Reaktionszone abgezogen wird. Auf diese Weise wird der Anteil der nicht umgesetzten Säure in dem abgezogenen, an Ester reichen flüssigen Produkt minimiert. Die Säure, der Alkohol und gegebenenfalls der Katalysator können mit Umgebungstemperatur oder vorgewärmt in die Reaktionszone eingeführt werden.

Aus der Reaktionszone werden überschüssiger Alkohol und entstandenes Reaktionswasser in Dampfform kontinuierlich entfernt. Die Alkohole können mit Wasser ein Azeotrop bilden. wie das bei Ethanol der Fall ist. Andere Alkohole, wie Methanol, führen Wasserdampf entsprechend dem Partialdruck des Wassers über dem Reaktionsgemisch mit. In beiden Fällen ist es zweckmäßig, die kontinuierliche Ausschleusung des Reaktionswassers mit einer ebenfalls kontinuierlichen Aufarbeitung des Alkohol-Wasser-Gemisches zu verbinden. wobei der Alkohol in die Reaktionszone zurückgeführt werden kann und das Wasser verworfen wird. Dies kann z.B. geschehen. indem man das Dampfgemisch unten in eine Kolonne einleitet. aus der man am Kopf praktisch reinen Alkohol (oder dessen Azeotrop mit Wasser) abzieht und diesen in die Reaktionszone zurückführt, während gleichzeitig ein wasserreicher Seitenstrom abgezogen wird, der getrennt aufgearbeitet oder auch verworfen, z.B. verbrannt werden kann. Aus der Reaktionszone wird weiterhin kontinuierlich ein flüssiges Reaktionsprodukt abgezogen, das reich an dem gebildeten Ester ist. aber auch nicht umgesetzte Säure und etwaige höhersiedende Nebenprodukte sowie Alkohol und kleine Mengen Wasser enthält. Dieses flüssige Reaktionsprodukt kann durch Destillation in seine Bestandteile zerlegt und der gewünschte Ester in hoher Reinheit gewonnen werden. Dies kann absatzweise geschehen, z.B. indem man zunächst alle flüchtigen Produkte von den nicht oder praktisch nicht destillierbaren Anteilen abtrennt und dann aus den flüchtigen Produkten durch fraktionierende Reindestillation den Ester gewinnt. Bei größeren Produktmengen lohnt sich eine kontinuierliche Aufarbeitung.

Die Ausbeute an dem gewünschten Ester läßt sich noch steigern und der Gehalt der Säure im Endprodukt weiter vermindern, wenn man das abgezogene und/oder als Sumpfprodukt anfallende an Ester reiche flüssige Reaktionsgemisch vor der Reindestillation nachverestert. Dazu wird es in dem Alkohol gelöst, und die Lösung wird erneut den Veresterungsbedingungen unterworfen. Durch Reindestillation lassen sich aus dem Produkt der Nachveresterung hochreine, praktisch säurefreie Ester in guten Ausbeuten herstellen.

Das Verfahren nach der Erfindung - Veresterung und Nachveresterung - wird in Apparaten durchgeführt, wie sie in der Technik üblich sind.

Beispielsweise kann ein Kessel verwendet werden, der über Einrichtungen zur geregelten Heizung, zur geregelten Zufuhr von Ausgangsstoffen. zum geregelten Abzug von flüssigem Reaktionsgemisch, zur Standhaltung sowie zur Trennung des Wasser- und Alkoholdampfgemisches verfügt, das den Kessel oben verläßt, wie zuvor beschrieben. Gegebenenfalls muß die Apparatur auch unter vermindertem Druck betrieben werden können. Vorrichtungen zum Durchmischen des Veresterungsgemisches. z.B. ein Flügelrührer. sind zweckmäßig. An Stelle eines Kessels kann man auch eine entsprechend ausgestatte Füllkörperkolonne verwenden, in die man beispielsweise oben die zu veresternde Säure und gegebenenfalls den Katalysator und im Mittelteil den Alkohol einführt, während das an Ester reiche Reaktionsprodukt im unteren Teil der Kolonne abgezogen wird. Die Füllkörper sorgen dann für gute Durchmischung.

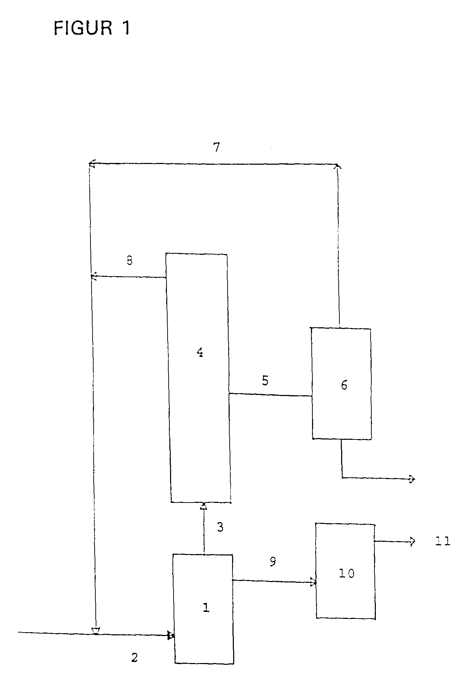

Das Blockschema einer geeigneten Apparatur zur Durchführung des Verfahrens der Erfindung ist in der Figur 1 dargestellt. In den unteren Teil des Reaktors **1** werden die zu veresternde Säure, der Alkohol und gegebenenfalls der Katalysator als Stoffstrom **2** eingeführt. Der im wesentlichen aus Alkohol- und Wasserdampf bestehende Dampfstrom **3** tritt unten in die Kolonne **4** ein, aus der als Seitenstrom **5** ein wasserreiches Alkohol-Wasser Gemisch austritt, das in der kontinuierlichen Destillation **6** auf Alkohol **7** aufgearbeitet wird. der ebenso wie der als Kopfstrom **8** aus der Kolonne **4** abgezogene praktisch reine Alkohol in den Stoffstrom **2** zurückgeführt wird. Der Produktstrom **9** wird dem Reaktor im oberen Teil entnommen, und aus diesem wird in dem Destillationsteil **10** der gewünschte Ester **11** gewonnen.

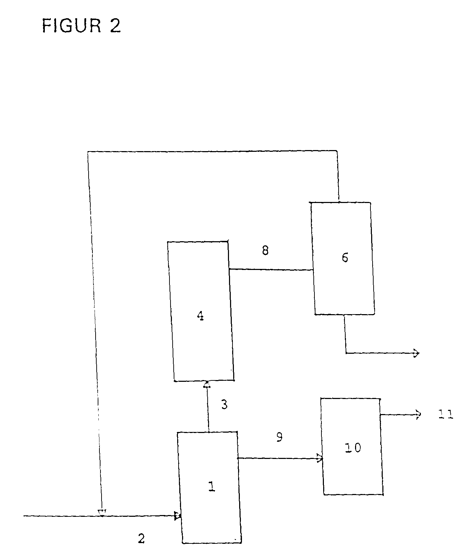

Eine vorteilhafte, vereinfachte Variante der Anordnung nach Figur 1 ist in der Figur 2 wiedergegeben. Dabei werden in der - dann entsprechend kürzeren - Kolonne **4** lediglich Ester und Säure von Alkohol und Wasser getrennt. Der Seitenstrom **5** entfällt, und der Kopfstrom **8** wird nicht unmittelbar in den Stoffstrom **2** zurückgeführt. sondern in der Kolonne **6** auf Alkohol aufgearbeitet. der dann in den Stoffstrom **2** zurückgeführt wird.

Das Verfahren der Erfindung wird in dem folgenden Beispiel erläutert. ist jedoch nicht auf diese spezielle Ausführungsform beschränkt. Alle Prozentangaben beziehen sich auf das Gewicht.

### Beispiel - 4-Chlorbuttersäuremethylester (CBME)

Rohe 4-Chlorbuttersäure (CBS) wurde in bekannter Weise hergestellt, indem in gamma-Butyrolakton (BL), das auf 100°C erwärmt wurde, solange gasförmiger Chlorwasserstoff eingeleitet wurde, bis dieser nicht mehr aufgenommen wurde. Danach wurde Stickstoff durch das Reaktionsgemisch geleitet, um überschüssigen Chlorwasserstoff zu verdrängen. Die Säurezahl lag danach bei 393. Aus 1.373g (15,8 Mol) BL wurden 1.818g rohe CBS erhalten.

Die rohe CBS wurde im Molverhältnis 6:1 mit Methanol verdünnt, also mit 3.037g Methanol (Lösung 1). Für die Veresterung wurden 2.211 g der Lösung 1 eingesetzt, entsprechend ca. 7,2 Mol BL. Die zur Veresterung verwendete Apparatur entspricht der Figur 1 und besteht aus
- einem doppelwandigen, beheizbaren Glaskolben mit einem Inhalt von 21 (Reaktor **1**)
- einem Einleitungsrohr für ein Gemisch aus CBS, Methanol und Katalysator (Stoffstrom **2**),
- einem Eintauchrohr für den Abzug des an Ester reichen Reaktionsgemisches (Produktstrom **9**),
- einem Rührer,
- einem 10 cm langen unteren und einem 20 cm langen oberen Kolonnenschuß mit Berlsätteln als Füllkörper (zusammen Kolonne **4**),
- einem Seitenabzug zwischen den beiden Kolonnenschüssen für den Abzug des wasserreichen Alkohol-Wasser-Gemisches (Seitenstrom **5**).
- einen Abzug für den Alkohol (Kopfstrom **8**) am Kopf der Kolonne,
- eine Destillationsbrücke zur Aufarbeitung des abgezogenen Reaktionsgemisches **9** (Destillationsteil **10**)

### 1. Herstellung eines Veresterungsgemisches

300g der Lösung 1 wurden im Reaktor vorgelegt, der Druck wurde auf 500mbar abgesenkt und die Lösung 1 zum Sieden am Rückfluß erhitzt. Innerhalb von 5 Stunden wurden weitere 456g der Lösung 1 zudosiert. Die Temperatur im Reaktionsgemisch stieg in dieser Zeit von 54 auf 72°C an. Eine Stunde nach Beginn der Zudosierung wurde mit dem Abzug am Kopf (Kopfstrom **8**) und zwischen den Kolonnenschüssen (Seitenstrom **5**) begonnen, wobei ein Rückflußverhältnis von 5:1 eingestellt wurde. Die als Seitenstrom **5** abgezogene Menge wurde durch ein Regulierventil geregelt. Innerhalb des Zeitraumes von 5 Stunden wurden erhalten:

| | |
|---|---|
| 234g | Kopfstrom 8 mit 0,44% Wasser |
| 143g | Seitenstrom 5 mit 2,04% Wasser |

### 2. Veresterungsphase

Die Stoffbilanz wurde für zwei Zyklen ermittelt. Im ersten Zyklus wurden innerhalb von 7 Stunden 825g Lösung 1 zudosiert. Ein Kopfstrom **8** und zwischen den Kolonnenschüssen ein Seitenstrom **5** wurden von Beginn an abgezogen; das Rückflußverhältnis betrug 2.5:1. Nach 5 Stunden war die Temperatur im Reaktionsgemisch von 72°C auf 92°C angestiegen. Zu diesem Zeitpunkt wurde mit der Abnahme von Reaktionsgemisch (Produktstrom **9**) begonnen. Innerhalb der 7 Stunden wurden erhalten:

| | |
|---|---|
| 201,4g | Kopfstrom **8** mit 1,0% Wasser |
| 299,7g | Seitenstrom **5** mit 18,1% Wasser |
| 32,6g | Produktstrom **9** |

Im zweiten Zyklus wurden innerhalb von 5 Stunden 630g Lösung 1 zudosiert. Das Rücklaufverhältnis betrug weiterhin 2,5:1. die Temperatur im Reaktionsgemisch 88 bis 92°C. Reaktionsgemisch (Produktstrom **9**) wurde von Anfang an abgezogen. Innerhalb der 5 Stunden fielen an:

| | |
|---|---|
| 115g | Kopfstrom **8** mit 0.4% Wasser |
| 240g | Seitenstrom **5** mit 11% Wasser |
| 83g | Produktstrom **9** |

Nach 5 Stunden wurde die Veresterung beendet. Im Reaktor **1** waren 805g Reaktionsgemisch (Sumpfprodukt) verblieben.

Insgesamt waren 2.211g Lösung eingesetzt worden. Zurückgewonnen wurden 2.140g. Der Verlust beträgt demnach 3.2%.

Die verschiedenen Ströme und Produkte hatten nach GC die folgende Zusammensetzung:

| Komponente | Kopfstrom **8** | Seitenstrom **5** | Produktstrom **9** | Sumpfprodukt |
|---|---|---|---|---|
| Methanol | 99,4 | 83,2 | 5,2 | 2,6 |
| Wasser | 0,5 | 12,7 | 2,4 | 1,2 |
| CBME | <0,1 | 4,0 | 70,3 | 74,8 |
| CBS | - | <0,1 | 4,3 | 3,2 |
| BL | <0,1 | <0,1 | 8,0 | 9,4 |

Die Tabelle zeigt. daß das Reaktionswasser praktisch vollständig im Seitenstrom **5** enthalten war und als Kopfstrom **8** praktisch reines Methanol anfiel.

### 3. Nachveresterung

Zur weiteren Verminderung des Gehalts an CBS wurden das Produkt des Produktstroms **9** und das Sumpfprodukt vereinigt (862g Rohester) und zusammen nachverestert. Der Rohester hatte nach GC die folgende Zusammensetzung (%):

| | |
|---|---|
| Methanol | 2,8 |
| Wasser | 1,2 |
| CBME | 73,6 |
| CBS | 3,5 |
| BL | 9,2 |

Der Rohester wurde mit der gleichen Menge Methanol verdünnt (Lösung 2) und so zur Nachveresterung eingesetzt. Dazu wurden im ersten Nachveresterungszyklus 400g Lösung 2 in den Reaktor gefüllt, es wurde ein Druck von 500mbar eingestellt. und die Lösung 2 wurde zum Rückfluß erhitzt. Innerhalb von 6 Stunden wurden 793g Lösung 2 zudosiert. Mit der Abnahme von Produkt am Kopf (Kopfstrom **8**)und zwischen den Kolonnenschüssen (Seitenstrom **5**) bei einem Rücklaufverhältnis von 2,5:1 wurde sofort begonnen. Reaktionsgemisch (Produktstrom **9**) wurde noch nicht abgezogen. Innerhalb der **6** Stunden wurden erhalten:

| | |
|---|---|
| 256g | Kopfstrom **8** mit 0,2% Wasser |
| 351g | Seitenstrom **5** mit 2.1% Wasser |

Im zweiten Nachveresterungszyklus wurden innerhalb von 4,5 Stunden 578g Lösung 2 zudosiert. Die Temperatur im Reaktionsgemisch betrug 92°C. das Rücklaufverhältnis wiederum 2.5:1. Mit der Abnahme von Reaktionsgemisch (Produktstrom **9**) wurde sofort begonnen. Während der 4,5 Stunden wurden erhalten:

| | |
|---|---|
| 73g | Kopfstrom **8** mit 0.3% Wasser |
| 179g | Seitenstrom **5** mit 3.2% Wasser |
| 104g | Produktstrom **9** |

Die Nachveresterung wurde beendet. Im Reaktor verblieben 779g Reaktionsgemisch (Sumpfprodukt).

Insgesamt wurden 1.742g Produkt als verschiedene Ströme und als Sumpfprodukt zurückgewonnen. Eingesetzt waren 1.771g Lösung 2. Der Verlust betrug 1,6%.

Die Produkte aus den verschiedenen Strömen (beide Zyklen vereinigt) und aus dem Sumpf hatten die folgende Zusammensetzung:

| Komponente | Kopfstrom **8** | Seitenstrom **5** | Produktstrom **9** | Sumpfprodukt |
|---|---|---|---|---|
| Methanol | 99,7 | 97,6 | 3,7 | 2,8 |
| Wasser | 0,1 | 2,0 | 0,5 | 0,4 |
| CBME | <0,1 | 0,3 | 78,1 | 80,1 |
| CBS | - | - | 0,6 | 0,5 |
| BL | <0,1 | <0,1 | 8,9 | 9,4 |

Durch die Nachveresterung konnten die Gehalte an CBS und Wasser im Produktstrom **9** und im Sumpfprodukt auf <1% gesenkt werden.

### 4. Reindestillation

Produktstrom 9 und das Sumpfprodukt wurden vereinigt (883g). Davon wurden 853g zur Reindestillation über eine 0,5m lange, mit Berlsätteln (4x4mm) gefüllten Destillationskolonne eingesetzt. Es wurden 4 Fraktionen abgenommen:

| Fraktion Nr. | Temperatur, °C | | Druck, mbar | Gewicht, g | Rücklaufverh. |
|---|---|---|---|---|---|
| | Sumpf | Kopf | | | |
| 1 | 81-115 | 38-107 | 133 | 9 | 20:1 |
| 2 | 115-125 | 108-110 | 133 | 632 | 5:1 |
| | 144 | | | | 20:1 |
| 3 | 145-147 | 115-131 | 133 | 37 | 20:1 |
| 4 | 154-229 | 135-130 | 133 | 83 | 5:1 |
| Rückstand | | | | 53 | |
| Kühlfalle | | | | 22 | |
| | Insgesamt : 836 | | | | |

Die Fraktionen hatten die folgende Zusammensetzung:

| Komponente | Fraktionen | | | |
|---|---|---|---|---|
| | 1 | 2 | 3 | 4 |
| Methanol | 2,3 | <0,1 | <0,1 | <0,1 |
| Wasser | 2,4 | <0,1 | <0,1 | <0,1 |
| CBME | 84,9 | 98,4 | 81,9 | 6,2 |
| CBS | 8,4 | <0,1 | <0,1 | <0,1 |
| BL | 0,3 | 1,5 | 17,5 | 93,5 |

Die Fraktion 3 enthält keine störenden Verunreinigungen und kann bei der nächsten Destillation wieder eingesetzt werden. Deshalb wird die in dieser Fraktion enthaltene Menge CBME bei der Ausbeuteberechnung mitberücksichtigt. Die Ausbeute an CBME (aus den Fraktionen 2 und 3) beträgt dann 71% d.Th., bezogen auf eingesetzes BL. Mit der Fraktion 4 wurde wieder einsetzbares BL zurückgewonnen. Die Ausbeute an CBME beträgt 82% d.Th., bezogen auf verbrauchtes BL.

## Patentansprüche

1. Verfahren zur kontinuierlichen Herstellung von Estern der Cyanessigsäure, 4-Chlorbuttersäure, Thioglykolsäure und Malonsäure durch Veresterung der Säuren mit Alkoholen, gegebenenfalls in Anwesenheit eines Katalysators, dadurch gekennzeichnet, daß man die Veresterung bei Temperaturen von 80 °C bis 130 °C im wesentlichen in nur einer Reaktionszone durchführt, der man die Säure und einen Überschuß an Alkohol kontinuierlich zuführt und aus der man Alkohol zusammen mit Reaktionswasser in Dampfform sowie ein an Ester reiches Reaktionsgemisch in flüssiger Form kontinuierlich entfernt und aus diesem den Ester gewinnt.

2. Verfahren nach Anspruch 1, dadurch gekennzeichnet, daß man die Saure und den Alkohol gemeinsam-der Reaktionszone an einer Stelle zuführt, die von der Stelle entfernt ist, an der das an Ester reiche flüssige Produkt der Reaktionszone entnommen wird.

3. Verfahren nach Anspruch 1 oder 2, dadurch gekennzeichnet, daß man die aus der Reaktionszone entweichenden Dämpfe des überschüssigen Alkohols und des Reaktionswassers in den unteren Teil einer Kolonne leitet, in deren mittlerem Teil man ein wasserreiches flüssiges Gemisch abzieht, das in einer weiteren Kolonne in Alkohol, der in die Reaktionszone zurückgeführt wird, und in Wasser getrennt wird, und in deren oberem Teil man praktisch reinen Alkohol abzieht, der in die Reaktionszone zurückgeführt wird.

4. Verfahren nach Anspruch 1 oder 2, dadurch gekennzeichnet, daß man die aus der Reaktionszone entweichenden Dämpfe des überschüssigen Alkohols und des Reaktionswassers in den unteren Teil einer Kolonne leitet, in deren oberem Teil man ein flüssiges, Alkohol und Wasser enthaltendes Gemisch abzieht, das in einer weiteren Kolonne in Alkohol, der in die Reaktionszone zurückgeführt wird, und Wasser getrennt wird.

5. Verfahren nach einem der Ansprüche 1 bis 4, dadurch gekennzeichnet, daß man aus dem an Ester reichen flüssigen Reaktionsgemisch durch Destillation den Ester gewinnt.

6. Verfahren nach einem der Ansprüche 1 bis 5, dadurch gekennzeichnet, daß der Alkohol ein Alkanol mit 1 bis 3 Kohlenstoffatomen ist.

7. Verfahren nach einem der Ansprüche 1 bis 6, dadurch gekennzeichnet, daß bei 4-Chlorbuttersäure die Temperatur in der Reaktionszone 80 bis 100 °C, vorteilhaft 85 bis 95 °C beträgt.

8. Verfahren nach einem der Ansprüche 1 bis 7, dadurch gekennzeichnet, daß das Molverhältnis von Alkohol zu Säure 15:1 bis 3:1, vorteilhaft 10:1 bis 4:1 und insbesondere 8:1 bis 5:1 beträgt.

## Revendications

1. Procédé de production en continu d'esters d'acide cyanacétique, d'acide 4-chlorobutyrique, d'acide thioglycolique, et d'acide malonique, par estérification des acides avec des alcools, éventuellement en présence d'un catalyseur,
caractérisé en ce qu'
on effectue l'estérification à des températures allant de 80 à 130°C, essentiellement dans seulement une zone de réaction, dans laquelle on amène l'acide et un excès d'alcool d'une manière continue et desquels on élimine en continu l'alcool conjointement avec l'eau de réaction sous forme vaporisée ainsi qu'un mélange réactionnel riche en ester sous forme liquide et à partir de celui-ci on obtient l'ester.

2. Procédé selon la revendication 1,
caractérisé en ce qu'
on amène l'acide et l'alcool conjointement à la zone de réaction à un emplacement éloigné de celui où le produit liquide riche en ester est retiré de la zone de réaction.

3. Procédé selon la revendication 1 ou la revendication 2,
caractérisé en ce qu'
on conduit les vapeurs qui s'échappent de la zone de réaction de l'alcool en excès et de l'eau de réaction, dans la partie inférieure d'une colonne, on soutire dans sa partie moyenne un mélange liquide riche en eau, qui est séparé dans une autre colonne, en alcool ramené à la zone de réaction et en eau, et on soutire dans sa partie supérieure de l'alcool pratiquement pur ramené à la zone de réaction.

4. Procédé selon la revendication 1 ou la revendication 2,
caractérisé en ce qu'
on conduit les vapeurs qui s'échappent de la zone de réaction, d'alcool en excès et d'eau de réaction dans la partie inférieure d'une colonne, on soutire dans sa partie supérieure un mélange liquide contenant de l'alcool et de l'eau, dont on sépare dans une autre colonne, l'eau de l'alcool, que l'on ramène dans la zone de réaction.

5. Procédé selon l'une des revendications 1 à 4,
caractérisé en ce qu'
on produit à partir du mélange réactionnel liquide riche en ester, l'ester par distillation.

6. Procédé selon l'une des revendications 1 à 5,
caractérisé en ce que
l'alcool est un alkanol ayant de 1 à 3 atomes de carbone.

7. Procédé selon l'une des revendications 1 à 6,
caractérisé en ce que
pour l'acide 4-chlorobutyrique, la température dans la zone de réaction s'élève de 80 à 100°C, avantageusement de 85 à 95°C.

8. Procédé selon l'une des revendications 1 à 7,
caractérisé en ce que
le rapport molaire de l'alcool à l'acide s'élève de 15:1 à 3:1, d'une manière avantageuse de 10:1 à 4:1 et en particulier de 8:1 à 5:1.

## Claims

1. A process for the continuous preparation of esters of cyanoacetic acid, 4-chlorobutyric acid, thioglycolic acid and malonic acid by esterification of the acids with alcohols, in the presence or absence of a catalyst, characterized in that the esterification is carried out at temperatures of from 80°C to 130°C essentially in only one reaction zone to which the acid and an excess of alcohol are continuously supplied and from which alcohol together with reaction water are continuously removed in vapor form and an ester-rich reaction mixture is continuously removed in liquid form and the ester is isolated from this.

2. A process according to claim 1, characterized in that the acid and the alcohol are fed together to the reaction zone at a point which is remote from the point at which the ester-rich liquid product is taken off from the reaction zone.

3. A process according to claim 1 or 2, characterized in that the vapors of the excess alcohol and the reaction water escaping from the reaction zone are passed into the lower part of a column, in the middle part of which a water-rich liquid mixture is taken off which is separated in a further column into alcohol, which is recycled to the reaction zone, and water, and in the upper part of which virtually pure alcohol is taken off, which is recycled to the reaction zone.

4. A process according to claim 1 or 2, characterized in that the vapors of the excess alcohol and the reaction water escaping from the reaction zone are passed into the lower part of a column, in the upper part of which a liquid alcohol- and water-containing mixture is taken off, which is separated in a further column into alcohol, which is recycled to the reaction zone, and water.

5. A process according to one of claims 1 to 4, characterized in that the ester is produced from the ester-rich liquid reaction mixture by distillation.

6. A process according to any one of claims 1 to 5, characterized in that the alcohol is an alkanol having from 1 to 3 carbon atoms.

7. A process according to any one of claims 1 to 6, characterized in that, in the case of 4-chlorobutyric acid, the temperature in the reaction zone is from 80 to 100°C, advantageously from 85 to 95°C.

8. A process according to any one of claims 1 to 7, characterized in that the molar ratio of alcohol to acid is from 15:1 to 3:1, advantageously from 10:1 to 4:1 and in particular from 8:1 to 5:1.
